# EUROPÄISCHE PATENTANMELDUNG

(11) **EP 1 568 788 A2**
(43) Veröffentlichungstag der Anmeldung: **31.08.2005**
(21) Anmeldenummer: 05003622.7
(22) Anmeldetag: 19.02.2005
(51) Int. Cl.: C14C 3/22, C07C 309/34

(54) **Aromatische Sulfonsäuren, ihr Herstellungsverfahren, ihre Aldehydkondensate und ihre Verwendung im Leder-, Papier- und Textilbereich**

(30) Priorität: 25.02.2004 DE 102004009096
(71) Anmelder: LANXESS Deutschland GmbH, 51368 Leverkusen (DE)
(72) Erfinder: Brinkmann, Nils, Dr., 51373 Leverkusen (DE); Kleban, Martin, Dr., 51381 Leverkusen (DE); Markgraf, Kirsten, Dr., 69469 Weinheim (DE)

(57) **Zusammenfassung**

Aromatische Hydroxysulfonate der Formel I 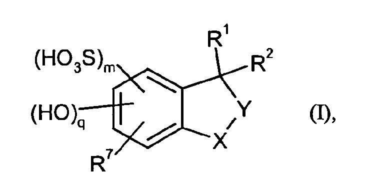 ihr Herstellungsverfahren, ihre Aldehydkondensate und ihr Verwendung im Leder-, Papier- und Textilbereich.

## Beschreibung

Die Erfindung betrifft aromatische Sulfonsäuren oder deren Salze, deren Aldehydkondensate, jeweils Verfahren zu deren Herstellung, ihre Verwendung als Lederhilfsmittel, insbesondere als Gerb- und Nachgerbstoffe, sowie die damit gegerbten und nachgegerbten Leder und Pelze.

Die Herstellung von Leder und Pelzen aus Häuten und Fellen verläuft in der Regel in mehreren Schritten. Nach den vorbereitenden Schritten der Wasserwerkstatt, wie Enthaaren, Entfleischen, Entkälken und Beizen besteht eine typische Abfolge aus Gerbung, Nachgerbung, Färbung, Fettung und Zurichtung. Die einzelnen Arbeitsschritte lassen sich dabei noch in weitere Untereinheiten aufteilen.

Während die Gerbung zu einer Erhöhung der Schrumpftemperatur des Leders führt, hat die Nachgerbung darauf kaum Einfluss. Unter Nachgerbung versteht man die Nachbehandlung von vorgegerbtem, oft chromgegerbtem Leder, um Farbe, Egalität, Weichheit, Fülle sowie das Verhalten gegen Wasser (Hydrophobie) zu optimieren und Gerbstoffe zu fixieren. Besonders die Gerbung, Nachgerbung und Färbung werden dabei gewöhnlich in verschiedenen sogenannten Gerbfässern unter Verwendung wässriger Gerbstoff-/Nachgerbstoff-Lösungen oder -Dispersionen respektive Farbstofflösungen durchgeführt.

DE-A-195 47 212 beschreibt polymere Polysulfonsäuren als Lederadditive, erhältlich durch Polymerisation von Monomeren ausgewählt aus der Gruppe, bestehend aus C₈-C₂₀-vinylaromatischen Verbindungen, Inden und Inden-Derivaten der allgemeinen Formel 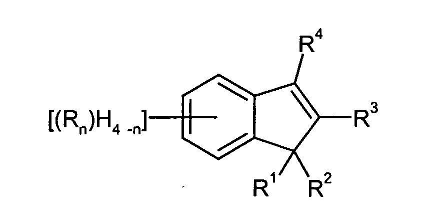 in der R für C₁-C₆-Alkyl steht und n eine ganze Zahl von 0 bis 3 bedeutet und R¹ bis R⁴ für Wasserstoff oder C₁-C₆-Alkyl steht, in Gegenwart von Schwefeltrioxid.

Aus EP-A-470 465 sind bereits aromatische Hydroxysulfonate bekannt. Diese weisen aber insbesondere hinsichtlich der Weichheit und Fülle des mit ihnen nachgegerbtem Leder noch verbesserungsbedürftige Eigenschaften auf.

Aufgabe der vorliegenden Erfindung war es daher, Gerb- und Nachgerbstoffe bereitzustellen, die die beschriebenen Nachteile des Stands der Technik nicht haben und sich insbesondere gut für schwarz zu färbendes Leder eignen.

Es wurden nun Verbindungen gefunden, die in Form ihrer Säure der Formel I entsprechen 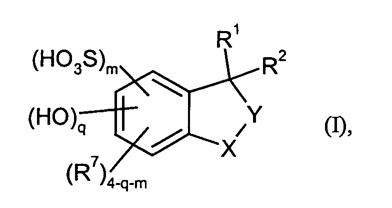 worin
X und Y gemeinsam für einen Rest der Formel 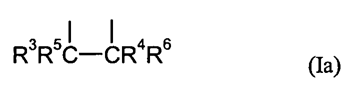 oder 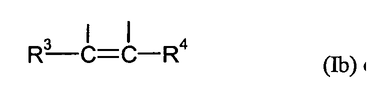 oder 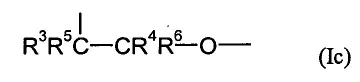 stehen, wobei
- Y: das R³-substituierte C-Atom enthält, d.h. das R³-substituierte C-Atom mit dem R¹-substituierten C-Atom verknüpft ist,
- R¹ bis R⁶: unabhängig voneinander für Wasserstoff, Alkyl-, insbesondere gegebenenfalls substituiertes C₁-C₄-Alkyl oder Aryl, insbesondere gegebenenfalls substituiertes Phenyl steht, wobei wenigstens einer der Reste R² bis R⁵ für einen hydroxysubstituierten gegebenenfalls mit n Sulfogruppen substituierten Arylrest steht, insbesondere für ein Hydroxyphenyl, vorzugsweise für sulfoniertes Hydroxyphenyl steht, wobei R⁵ und R⁶ zusätzlich gegebenenfalls auch unabhängig voneinander für Hydroxy stehen,
- R⁷: für Wasserstoff oder den Rest 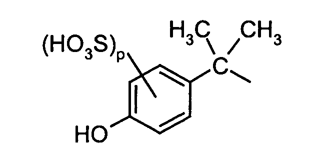
steht, wobei im Falle von mehreren Resten R⁷ diese Bedeutungen unabhängig voneinander gelten,
- m: für eine Zahl von 0 bis 3 steht,
- n: für eine Zahl von 0 bis 4 steht,
- p: für eine Zahl von 0 bis 4 steht,
- q: für 0 oder 1 steht,
wobei die Summe aus m, n und p wenigstens 1, bevorzugt 1,3 bis 4, besonders bevorzugt 1,8 bis 3,2 beträgt.

Als mögliche Substituenten der Alkylreste kommen beispielsweise in Frage: Hydroxy, Amino, C₆-C₁₀-Aryl, C₁-C₄-Alkoxy oder Carboxy.

Als bevorzugte Alkylreste kommen beispielsweise in Frage: Methyl, Ethyl, Propyl, n-Butyl, IsoButyl, Pentyl, insbesondere Methyl und Ethyl.

Als mögliche Substituenten der Arylreste kommen beispielsweise in Frage: C₁-C₄-Alkyl und/oder Hydroxy, und/oder Cycloalkyl, und/oder C₁-C₄-Alkoxy, und/oder C₁-C₄-Hydroxyalkoxy-, und/oder Aroxy-, und/oder Aralkoxy-, und/oder Carboxy-, und/oder Carbalkoxy-Reste, insbesondere C₁-C₄-Alkyl und/oder Hydroxy.

Bevorzugt sind Verbindungen der Formel Ia oder I c, insbesondere solche, in denen R² und/oder R³ und/oder R⁴ für hydroxysubstituiertes Phenyl steht, das gegebenenfalls jeweils n Sulfogruppen enthält, wobei n sowie die übrigen Substituenten die oben genannten Bedeutungen besitzen. Besonders bevorzugt sind solche Verbindungen der Formel Ia, die in Form ihrer Säure der Formel II oder III entsprechen und Verbindungen der Formel Ic, die in Form ihrer Säure der Formel IV oder V entsprechen. 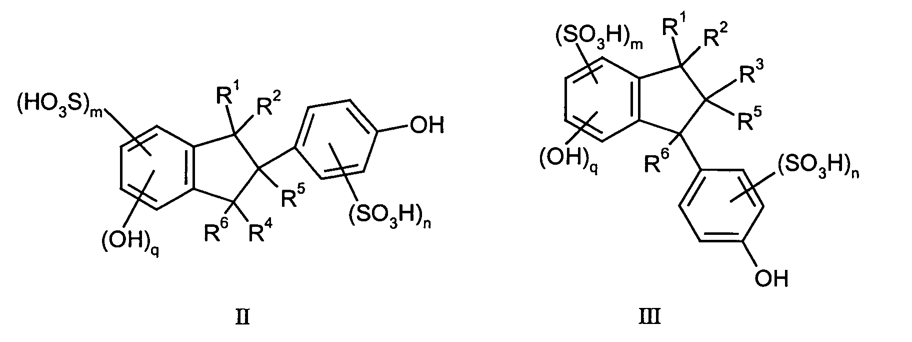 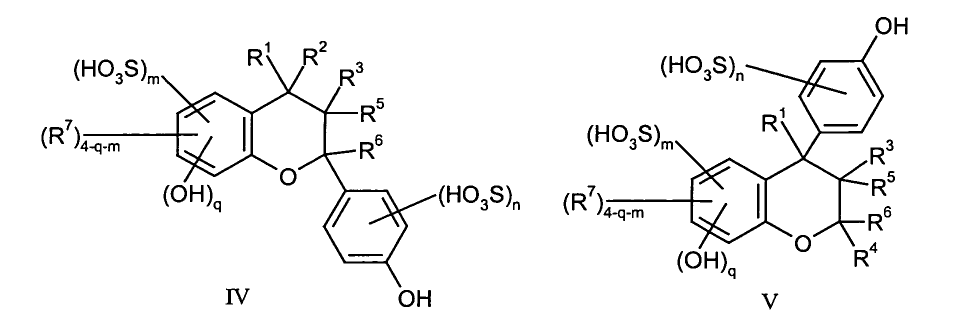 wobei
- R¹ bis R⁶: soweit vorhanden, jeweils unabhängig voneinander für Wasserstoff, Alkyl, vorzugsweise C₁-C₄-Alkyl, insbesondere Methyl oder Aryl, vorzugsweise gegebenenfalls mit n Sulfogruppen sulfoniertes Hydroxyphenol stehen, bevorzugt für Wasserstoff, Methyl oder Hydroxyphenyl stehen, und
- R⁷: für Wasserstoff oder den Rest 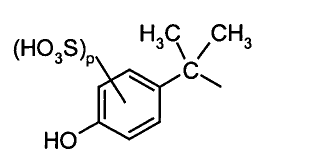
steht, wobei im Falle von mehreren Resten R⁷ diese Bedeutungen unabhängig voneinander gelten,
- m: eine Zahl von 0 bis 3 bedeutet und
- n: eine Zahl von 0 bis 4 bedeutet,
- p: eine Zahl von 0 bis 4 bedeutet,
- q: für 0 oder 1 steht,
wobei die Summe aus m, n und p wenigstens für 1, bevorzugt 1,3 bis 4, besonders bevorzugt 1,8 bis 3,2 steht.

In einer weiteren bevorzugten Ausführungsform entspricht die Formel Ia bis Ic Verbindungen mit q = 1.

In einer weiter bevorzugten Ausführungsform entspricht die Formel Ia der Formel IIa oder IIIa und die Formel Ic der Formel IVa oder Va 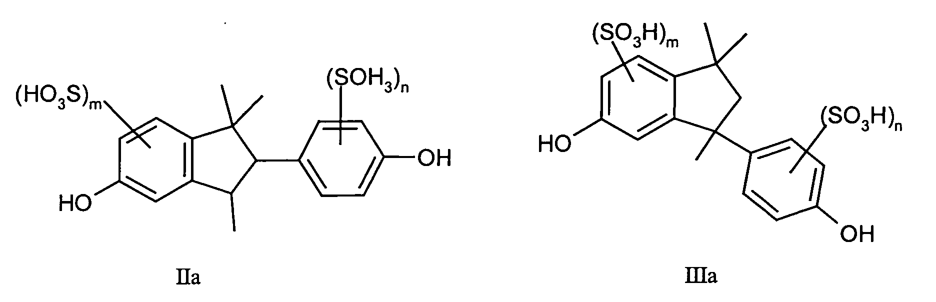 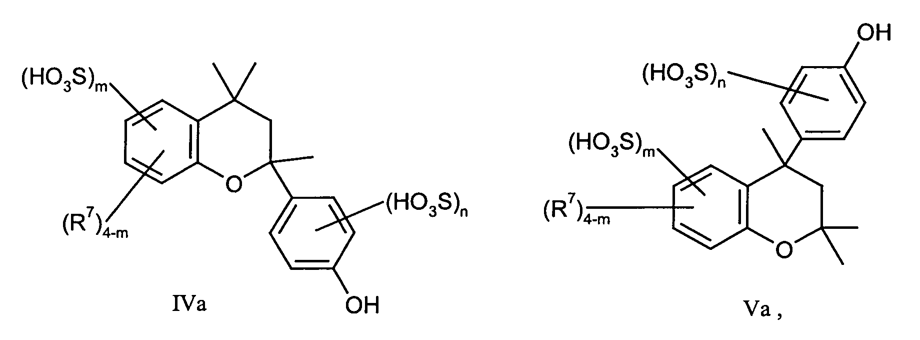 worin
R⁷ für Wasserstoff oder den Rest 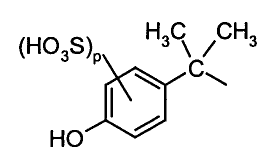 steht, wobei im Falle von mehreren Resten R⁷ diese Bedeutungen unabhängig voneinander gelten, und
m, n und p die oben angegebene Bedeutung besitzen.

Die in den Formeln als Säureformen beschriebenen Verbindungen liegen vorzugsweise als Salze, d.h. als Sulfonate vor. Als mögliche Gegenionen kommen vorzugsweise Alkali, Erdalkali oder Ammoniumverbindungen in Frage. Bevorzugt sind: Natrium, Lithium, Kalium, Magnesium, Ammonium, sowie die protonierten Formen des Triethanolamins, Diethanolamins oder Ethanolamins sowie Mischungen davon.

Die Erfindung betrifft weiterhin ein Verfahren zur Herstellung von Verbindungen der Formel I, das dadurch gekennzeichnet ist, dass man Verbindungen der Formel VI 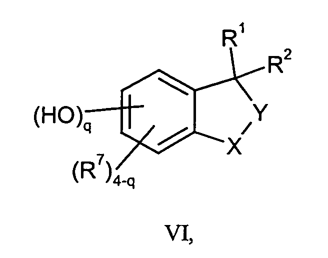 worin
- X, Y, R¹, R², R⁷ und q: die oben angegebene Bedeutung besitzen und wenigstens ein R⁷ für Wasserstoff steht,
mit einem Sulfonierungsmittel sulfoniert.

Bevorzugt haben die Reste der eingesetzten Verbindung der Formel VI auch sonst keine Sulfonsäuregruppen. Vorzugsweise entsprechen also die eingesetzten Verbindungen der Formel VI denen der Sulfongruppen-freien Formel I, d.h. vorzugsweise n = m = p = 0.

Das Ausgangsprodukt der Formel VI kann dabei als Reinstoff vorliegen, als Isomerengemisch oder als Mischung von Verbindungen, die der Definition von VI genügen. Bevorzugt sind Verbindungen der Formel VI die der Formel VIIa oder VIIb oder VIIc oder VIId entsprechen, 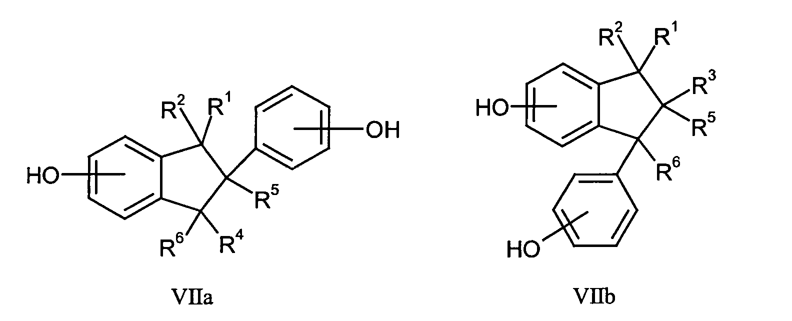 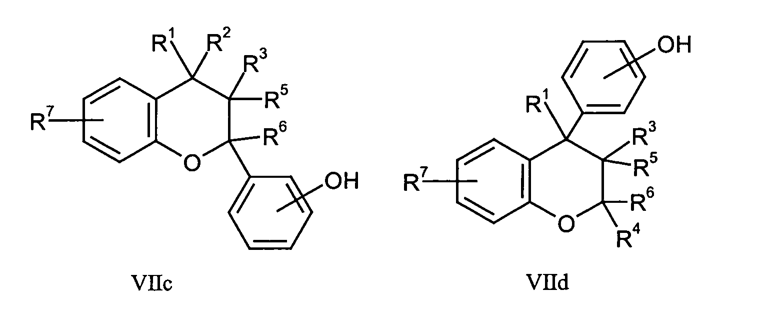 wobei
die Reste
- R¹ bis R⁷: jeweils unabhängig voneinander die obige Bedeutung haben können und bevorzugt keine Sulfonsäuregruppen tragen.

Besonders bevorzugt sind die Verbindungen der Formel VIIIa, VIIIb, VIIIc sowie VIIId

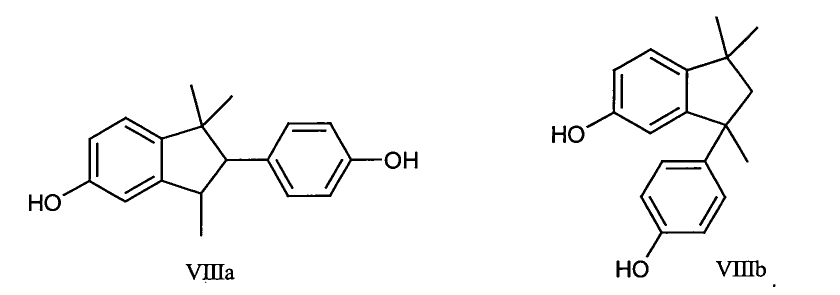

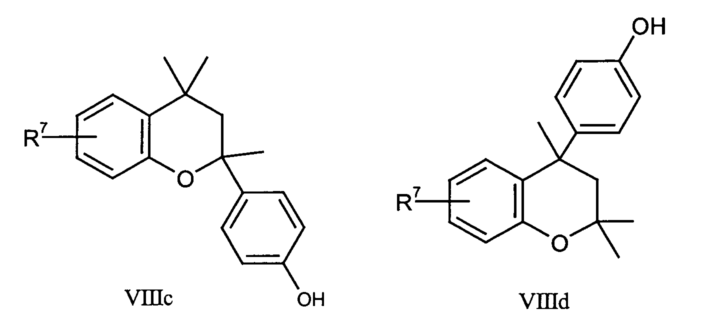

Die Sulfonierung des Ausgangsproduktes VI oder seiner Vorzugsformen ist beispielsweise möglich durch Umsetzung mit Schwefeltrioxid, Schwefelsäure oder Chlorsulfonsäure bevorzugt durch Anwendung von konzentrierter Schwefelsäure als Sulfonierungsmittel.

In einer bevorzugten Ausführungsform wird das Ausgangsprodukt erfindungsgemäß mit Schwefelsäure bei einer Temperatur von 50 bis 200°C, bevorzugt bei 80 - 150°C, besonders bevorzugt bei 90 - 130°C umgesetzt und nach erfolgter Sulfonierung vorzugsweise mit einer Base, bevorzugt einer Alkalihydroxyd- oder Erdalkalihydroxyd-Lösung oder einer Stickstoff-Base, besonders bevorzugt Ammoniak, auf einen pH-Wert von 1 bis 7, vorzugsweise von 2,5 bis 5 eingestellt.

Die so erhaltenen erfindungsgemäßen Verbindungen der Formel I können in wässriger Lösung verbleiben und eingesetzt werden oder einer Trocknung, besonders einer Sprühtrocknung unterzogen werden. Der Stoff weist eine rückstandslose Wasserlöslichkeit oder Wasseremulgierbarkeit bei 20°C von mehr als 50 g/L auf.

In einer bevorzugten Ausführungsform werden die Ausgangsverbindung VI oder ihre Vorzugsformen in Gegenwart anderer aromatischer Verbindungen wie beschrieben sulfoniert. Dabei kommen bevorzugt Naphthalin, Phenol, Dihydroxyphenylsulfon, Diarylether, 4,4'-Isopropylidendiphenol, o-[1-(4-Hydroxyphenyl)-1-methylethyl]phenol, Bisphenyl oder Terphenyl sowie deren Alkylderivate oder Mischungen davon in Frage. Bevorzugt haben die Ausgangsverbindungen der Formel VI im Gemisch mit anderen aromatischen Verbindungen einen Anteil von mehr als 15 Gew.- %, besonders bevorzugt mehr als 30 Gew.-% bezogen auf die Eduktmischung.

Ganz besonders bevorzugt wird eine Mischung enthaltend

| | |
|---|---|
| 0,5 bis 20 Gew.-% | VIIa, insbesondere VIIIa |
| 0,5 bis 20 Gew.-% | VIIb, insbesondere VIIIb |
| 2 bis 25 Gew.-% | VIc, insbesondere VIIIc |
| 2 bis 25 Gew.-% | VIId, insbesondere VIIId |
| 0,5 bis 20 Gew.-% | Phenol |
| 10 bis 50 Gew.-% | 4,4'-Isopropylidendiphenol und |
| 5 bis 40 Gew.-% | o-[1-(4-Hydroxyphenyl)-1-methylethyl]phenol |

eingesetzt, wobei die Mischung vorzugsweise aus mehr als 80 %, vorzugsweise mehr als 85 Gew.-% aus den oben mit Mengenbereichen versehenen Komponenten besteht.

Ganz besonders bevorzugt handelt es sich bei der eingesetzten Mischung um eine Mischung, wie sie beispielsweise anfällt bei Verfahren zur Herstellung von Bisphenolen durch säurekatalysierte Umsetzung von Phenolen mit Carbonylverbindungen. Diese Verfahren sind beispielsweise bekannt aus US-A 2 775 620 und aus EP-A-0 342 758. Ferner wird die Mischung, die zur Herstellung der erfindungsgemäßen Stoffe eingesetzt wird, auch beschrieben in der Patentanmeldung DE-A-100 15 864. DE 100 15 864 beschreibt die Verwendung eines Bisphenol A enthaltenden Stoffgemisches zur Herstellung von Polymerwerkstoffen.

Bevorzugt ist eine Mischung verschiedener Verbindungen der Formel I, insbesondere solche der Formel Ia und Ic. Erfindungsgemäß bevorzugt sind Mischungen von II, III, IV und V insbesondere IIa, IIIa, IVa und Va.

Mischungen enthaltend Verbindungen der Formel I können selbstverständlich noch andere Verbindungen enthalten. Bevorzugt ist der Anteil der erfindungsgemäßen Verbindungen der Formel I dabei größer 10 Gew.-%, insbesondere größer als 25 Gew.-%, bezogen auf die Mischung.

Die Erfindung betrifft somit auch Mischungen, enthaltend

| | |
|---|---|
| 0,5 bis 20 Gew.-% | II, insbesondere IIa |
| 0,5 bis 20 Gew.-% | III, insbesondere IIIa |
| 2 bis 25 Gew.-% | IV, insbesondere IVa |
| 2 bis 25 Gew.-% | V, insbesondere Va |
| 0,5 bis 20 Gew.-% | sulfoniertes Phenol |
| 10 bis 50 Gew.-% | sulfoniertes 4,4'-Isopropylidendiphenol und |
| 5 bis 40 Gew.-% | sulfoniertes o-[1-(4-Hydroxyphenyl)-1-methylethyl]phenol, |

wobei die Mischung vorzugsweise aus mehr als 80 Gew.-% vorzugsweise zu mehr als 85 Gew.-% aus den mit Mengenbereichen versehenen Komponenten besteht.

Bevorzugt liegt diese Mischung als wässrige Lösung vor.

Die Erfindung betrifft weiterhin aliphatische Aldehyd-Kondensate, besonders auf Basis von Formaldehyd, Acetaldehyd, Isobutyraldehyd, Propionaldehyd, Glutaraldehyd und Glyoxal, ganz besonders Formaldehyd-Kondensate auf Basis der erfindungsgemäßen Verbindungen der Formel I. "Auf Basis von" bedeutet, dass neben Aldehyden, insbesondere neben Formaldehyd und der oder den Verbindungen der Formel I noch weitere kondensierbare bzw. reagierende Verbindungen bei der Kondensatherstellung eingesetzt werden können. Als weitere kondensierbare Verbindungen sind beispielsweise weitere von I verschiedene aromatische Verbindungen zu verstehen, die gegebenenfalls sulfoniert sein können.

Als solche können beispielsweise genannt werden: Benzol, Phenol, Naphthalin, Biphenyl, Terphenyl, Bishydroxyphenylsulfon, Diarylether, 4,4'-Isopropylidendiphenol oder o-[1-(4-Hydroxyphenyl)-1-methylethyl]phenol sowie deren Sulfonsäuren oder Sulfonate. Weiterhin kommen in Frage obige Aromaten, die durch C₁-C₄-Alkyl- und/oder Hydroxy-, und/oder Cycloalkyl-, und/oder C₁-C₄-Alkoxy-, und/oder C₁-C₄-Hydroxyalkoxy-, und/oder Aroxy-, und/oder Aralkoxy-, und/oder Carboxy-, und/oder Carbalkoxy-Reste substituiert sind. Beispielhaft seien genannt: Phenol, Xylol, Mesitylen, Naphthalin, Diphenyl, Terphenyl, Kresole, 4,4'-Dihydroxydiphenylsulfon, β-Naphthol, Dihydroxybenzole, Resorcin, 4,4'-Isopropylidendiphenol, o-[1-(4-Hydroxyphenyl)-1-methylethyl]phenol, Diphenylether und Ditolylether.

Als weitere reagierende Verbindungen können beispielsweise Amine oder Amide, insbesondere solche der Kohlensäure wie beispielsweise Harnstoff, Melamin oder Dicyandiamid zur Herstellung der Kondensate mitverwendet werden.

Der Anteil der erfindungsgemäßen Verbindungen der Formel I zur Herstellung der erfindungsgemäßen Kondensate beträgt vorzugsweise mehr als 25 Gew.%, insbesondere mehr als 50 Gew.-%, bezogen auf die Summe aller eingesetzten aromatischen Verbindungen.

Ebenfalls bevorzugt ist es, zur Herstellung der erfindungsgemäßen Kondensationsprodukte die erfindungsgemäßen Mischungen einzusetzen.

Die erfindungsgemäßen Kondensationsprodukte liegen vorzugsweise wie die erfindungsgemäßen Verbindungen der Formel I als Salze vor, wobei als Gegenionen ebenfalls die oben genannten in Frage kommen. Bevorzugt weisen die erfindungsgemäßen Kondensationsprodukte einen Kondensationsgrad von 1 bis 7 auf.

Die Herstellung der erfindungsgemäßen Kondensate, erfolgt vorzugsweise analog der in EP-816 406 angegebenen Weise.

Die Erfindung betrifft weiterhin die Verwendung der erfindungsgemäßen Verbindungen der Formel I, Mischungen enthaltend Verbindungen der Formel I und/oder die auf Basis dieser Verbindungen hergestellten aliphatischen Aldehyd-Kondensate zum Gerben von Häuten oder Fellen oder zum Nachgerben mineralisch gegerbter Leder, insbesondere chromgegerbten Leder dadurch gekennzeichnet, dass die Häute oder Felle bzw. das gegerbte Leder mit den erfindungsgemäßen Stoffen in wässriger Flotte behandelt werden.

Ebenfalls Gegenstand der Erfindung sind Leder oder Pelze, welche mit den erfindungsgemäßen Verbindungen gegerbt oder nachgegerbt werden.

Der Einsatz von Gerb-/Nachgerbstoffen ist dem Fachmann bekannt und wird unter anderem auch beschrieben in Herfeld (Herausgeber) "Bibliothek des Leders", Bd. 3, S. 306-314, Beispiele 10-16, Umschau-Verlag. Eine Messung der Schrumpftemperatur (Gerbung) erfolgt beispielsweise nach der in DIN EN 12993 angegebenen Methode.

In einer bevorzugten Verwendung werden die erfindungsgemäßen Leder oder Pelze hergestellt, indem man in einer handelsüblichen Gerbapparatur wie Gerbfass, Mixer oder Dosamat handelsübliches Wet Blue auf einen pH-Wert von 4,0 bis 6,5, bevorzugt 4,8 bis 5,5 einstellt und anschließend in wässriger Flotte mit 3-20 % des erfindungsgemäßen Nachgerbstoffes allein oder in Kombination mit weiteren Nachgerbmitteln/Farbstoffen/Fettungsmitteln nachgerbt. Das so behandelte Leder kann betriebsüblich weiter verarbeitet werden.

Leder, welche mit dem erfindungsgemäßen Stoff gegerbt oder nachgegerbt werden, weisen neben einer Weichheit eine besondere Fülle auf. Daneben führt der erfindungsgemäße Stoff zu einer verbesserten Färbbarkeit.

Der erfindungsgemäße Stoff kann selbstverständlich auch in Verbindung mit weiteren Zusatzstoffen zur Anwendung kommen. Als solche kommen beispielsweise Neutralisationsmittel, Puffer, Dispergatoren, Entschäumer, Fette, Hydrophobiermittel, Färbereihilfsmittel oder andere Gerb-/Nachgerbstoffe in Frage.

Die Erfindung betrifft weiter die Verwendung des erfindungsgemäßen Stoffes als Hilfsmittel für die Papier- oder Textilanwendung.

Im Rahmen dieser Erfindung gelten auch alle Kombinationen der oben offenbarten allgemeinen Bereiche und der Vorzugsbereiche sowie der Vorzugsbereiche untereinander als offenbarte Vorzugsbereiche.

### Beispiele

### Beispiel 1a: Synthese eines erfindungsgemäßen sulfonierten Hydroxyaromaten

250 g der Komponente VIIIb werden auf 120°C erwärmt und im Laufe einer Stunde werden 408,8 g 96 %ige Schwefelsäure zugegeben. Bei einer Temperatur von 125°C wird 5 Stunden gerührt. Es resultiert eine klare, wasserlösliche, dunkelbraune Flüssigkeit (Zwischenprodukt I). Nach Zudosierung von 200 g Wasser wird auf 60°C abgekühlt und im Laufe einer Stunde wässrige Ammoniak-Lösung (25 %ig) zugegeben, bis ein pH-Wert von 3,0 resultiert. Die Einstellung eines Feststoffgehaltes von 50 % erfolgt durch weitere Zugabe von Wasser. Es resultiert eine niedrigviskose, dunkelbraune Lösung.

### Beispiel 1b: Synthese eines erfindungsgemäßen sulfonierten Hydroxyaromaten

250 g einer Mischung, bestehend zu mehr als 80 Gew.-% aus VIIIa (3 Gew.-%), VIIIb (9 Gew.-%), VIIIc (7 Gew.-%), VIIId (11 Gew.-%), Phenol (8 Gew.-%), 4,4'-Isopropylidendiphenol (42 Gew.-%) und o-[1-(4-Hydroxyphenyl)-1-methylethyl]phenol (20 Gew.-%) werden auf 110°C erwärmt. Im Laufe einer Stunde werden 410 g 96 %ige Schwefelsäure zugegeben. Bei einer Temperatur von 125°C wird 5 Stunden gerührt. Es resultiert eine klare, wasserlösliche, dunkelbraune Flüssigkeit (Zwischenprodukt I). Nach Zudosierung von 220 g Wasser wird auf 60°C abgekühlt und im Laufe einer Stunde wässrige Ammoniak-Lösung (25 %ig) zugegeben, bis ein pH-Wert von 3,0 resultiert. Die Einstellung eines Feststoffgehaltes von 50 % erfolgt durch weitere Zugabe von Wasser. Es resultiert eine niedrigviskose, dunkelbraune Lösung.

### Beispiel 1c: Synthese eines erfindungsgemäßen sulfonierten Hydroxyaromaten

250 g einer Mischung, bestehend zu mehr als 80 Gew.-% aus VIIIa (11 Gew.-%), VIIIb (13 Gew.-%), VIIIc (16 Gew.-%), VIIId (14 Gew.-%), Phenol (16 Gew.-%), 4,4'-Isopropylidendiphenol (17 Gew.-%) und o-[1-(4-Hydroxyphenyl)-1-methylethyl]phenol (13 Gew.-%) werden auf 120°C erwärmt und im Laufe einer Stunde werden 405 g 96 %ige Schwefelsäure zugegeben. Bei einer Temperatur von 100°C wird 5 Stunden gerührt. Es resultiert eine klare, wasserlösliche, dunkelbraune Flüssigkeit (Zwischenprodukt I). Nach Zudosierung von 210 g Wasser wird auf 60°C abgekühlt und im Laufe einer Stunde wässrige Ammoniak-Lösung (25 %ig) zugegeben, bis ein pH-Wert von 3,0 resultiert. Die Einstellung eines Feststoffgehaltes von 50 % erfolgt durch weitere Zugabe von Wasser. Es resultiert eine niedrigviskose, dunkelbraune Lösung.

### Beispiel 2: Synthese eines erfindungsgemäßen sulfonierten Hydroxyaromaten

250 g einer Mischung, bestehend zu mehr als 80 Gew.-% aus VIIIa (3 Gew.-%), VIIIb (9 Gew.-%), VIIIc (7 Gew.-%), VIIId (11 Gew.-%), Phenol (8 Gew.-%), 4,4'-Isopropylidendiphenol (42 Gew.-%) und o-[1-(4-Hydroxyphenyl)-1-methylethyl]phenol (20 Gew.-%) werden auf 110°C erwärmt und im Laufe einer Stunde werden 603,7 g 65 %ige Schwefelsäure zugegeben (schwach exotherme Reaktion). Unter vermindertem Druck (Enddruck 50 mbar) wurden 125 g Wasser abdestilliert. Bei einer Temperatur von 125°C wird 5 Stunden gerührt und anschließend auf 110°C abgekühlt. Es resultiert eine klare, wasserlösliche, dunkelbraune Flüssigkeit. Nach Zudosierung von 200 g Wasser wird auf 60°C abgekühlt und 374,6 g wässrige Ammoniak-Lösung (25 %ig) zugegeben. Durch Zugabe von Wasser wird ein Feststoffgehalt von 40 % eingestellt. Es resultiert ein sehr viskoses dunkelbraunes Produkt.

### Beispiel 3: Synthese eines erfindungsgemäßen Kondensats

Naphthalin wird mit 1,4 Äquivalenten H₂SO₄ bei 145°C für 2 h sulfoniert. Ein Gemisch aus 189 g der so erhaltenen Naphthalinsulfonsäuren, 129 g des Zwischenproduktes aus Beispiel I sowie 165 g 4,4'-Dihydroxyphenylsulfon und 81 g Formaldehyd-Lösung 37 % wird in 264 g Wasser bei 100 - 105°C für 2,5 h kondensiert. Das erhaltene Produkt wird mit Natronlauge und Phthalsäure auf pH 3,0 und Säurezahl (SZ) 81 eingestellt und sprühgetrocknet.

### Vergleichsbeispiel 1: Vergleichsprodukt IV

Phenol wird mit 2 Mol H₂SO₄ 2 h bei 120°C sulfoniert, abgekühlt, mit Natronlauge auf pH 6 gestellt und sprühgetrocknet. (EP-A 470 465 Beispiel 2)

### Vergleichsbeispiel 2: Vergleichsprodukt V

Ditolylether wird mit 1,7 äquivalenten Schwefelsäure 3 h bei 145°C sulfoniert, mit 0.7 Mol Formaldehyd 3 h kondensiert, abgekühlt, mit Natronlauge und Glutarsäure auf pH 3.5 und Säurezahl 50 gestellt und sprühgetrocknet.

### Anwendungsbeispiel 1: Nachgerbung

Wet Blue (Rind, 100 g) wird im Fass mit 100 % Wasser (Prozentangaben immer bezogen auf das Wet Blue-Gewicht), 1 % Natriumformiat und 0,8 % Natriumbicarbonat über Nacht bei 40°C bewegt. Nach Ablassen der Flotte und Spülen wird im Fass mit 100 % Wasser und 5 % des Gerbstoffes gemäß Beispiel 1 (die Prozentangabe bezieht sich auf den Feststoffgehalt) bei 40°C 2 Stunden bewegt. Schließlich werden die Leder mit 300 % Wasser, 0,5 % Farbstoff und 10 % einer handelsüblichen Fettmischung 90 Minuten bei 50 °C im Fass bewegt. Nach Zugabe von 2,5 % Ameisensäure (85 %ig) und weiterer Behandlung im Fass für 15 Minuten wird gespült, ausgestoßen und hängegetrocknet.

Man erhält ein Leder, das in Weichheit und Fülle dem mit den Vergleichsprodukten aus den Vergleichsbeispielen 1 und 2 erhaltenen deutlich überlegen ist. Das Leder ist darüber hinaus deutlich intensiver gefärbt als vergleichbare Leder, die mit Vergleichsbeispiel 2 behandelt wurde.

### Anwendungsbeispiel 2: Gerbung

Gewichtsangaben beziehen sich auf Blößengewicht.

Rindsblöße wird auf 3,0 mm gespalten, handelsüblich entkälkt, gebeizt und gepickelt. Zu der so vorbereiteten Haut wird bei pH 3,5 in 200 % Flotte 8 % eines handelsüblichen Vorgerbstoffes gegeben (Gemisch aus Naphthalinsulfonsäuren) und nach 2 h in zwei Raten 25 % Gerbstoff. Nach 36 h im Gerbfass bei 25 - 30°C wird eine Probe zur Bestimmung der Schrumpftemperatur nach DIN EN 12993 entnommen. Anschließend werden 2 % eines handelsüblichen Fettungsmittels auf Lanolin/-Fischöl-Basis zugegeben, 2 Stunden bewegt, die Flotte abgelassen und die Leder hängegetrocknet.

Die Messung der Schrumpftemperaturen ergibt:

| Produkt | Keines (Blöße) | Gerbstoff aus Beispiel 2 | Gerbstoff aus Beispiel 3 | Vergleichsbeispiel 1 | Vergleichsbeispiel 2 |
|---|---|---|---|---|---|
| Temperatur [°C] | 55 | 63 | 68 | 56 | 55 |

## Patentansprüche

1. Verbindungen die in Form ihrer Säure der Formel I entsprechen 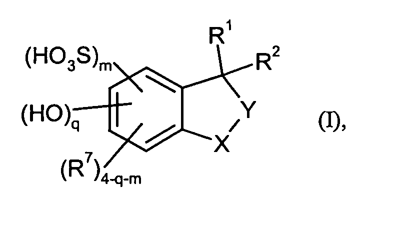 worin
X und Y gemeinsam für einen Rest der Formel 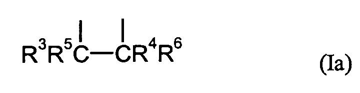 oder 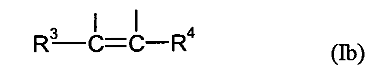 oder 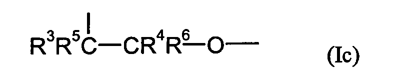 stehen, wobei
Y das R³-substituierte C-Atom enthält, d.h. das R³-substituierte C-Atom mit dem R¹-substituierten C-Atom verknüpft ist,
R¹ bis R⁶ unabhängig voneinander für Wasserstoff, Alkyl-, insbesondere gegebenenfalls substituiertes C₁-C₄-Alkyl oder Aryl, insbesondere gegebenenfalls substituiertes Phenyl steht, wobei wenigstens einer der Reste R² bis R⁵ für einen hydroxysubstituierten gegebenenfalls mit n Sulfogruppen substituierten Arylrest steht, insbesondere für ein Hydroxyphenyl, vorzugsweise für sulfoniertes Hydroxyphenyl steht, wobei R⁵ und R⁶ zusätzlich gegebenenfalls auch für Hydroxy stehen,
R⁷ für Wasserstoff oder den Rest 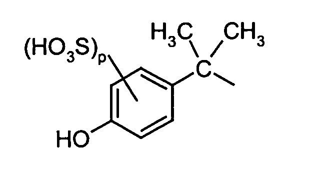
steht, wobei im Falle von mehreren Resten R⁷ diese Bedeutungen unabhängig voneinander gelten,
m für eine Zahl von 0 bis 3 steht,
n für eine Zahl von 0 bis 4 steht,
p für eine Zahl von 0 bis 4 steht,
q für 0 oder 1 steht,
wobei die Summe aus m, n und p wenigstens 1, bevorzugt 1,3 bis 4, besonders bevorzugt 1,8 bis 3,2 beträgt.

2. Verbindungen gemäß Anspruch 1, **dadurch gekennzeichnet, dass** X und Y gemeinsam für einen Rest der Formel (Ia) oder (Ic) stehen.

3. Verbindungen gemäß wenigstens einem der Ansprüche 1 oder 2, **dadurch gekennzeichnet, dass** sie in Form ihrer Säure der Formel II, III, IV oder V entsprechen, 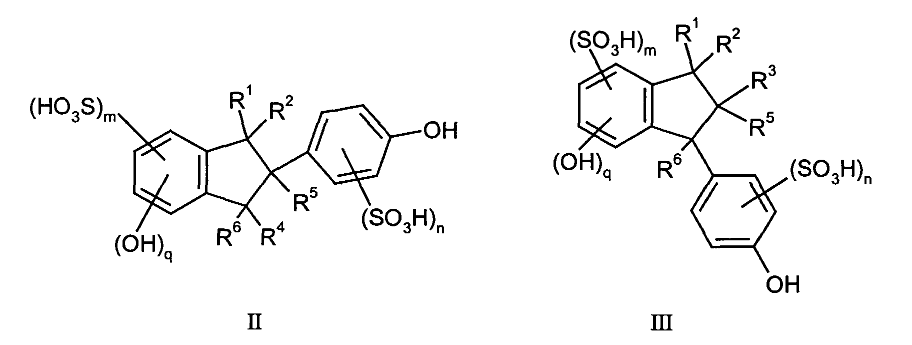 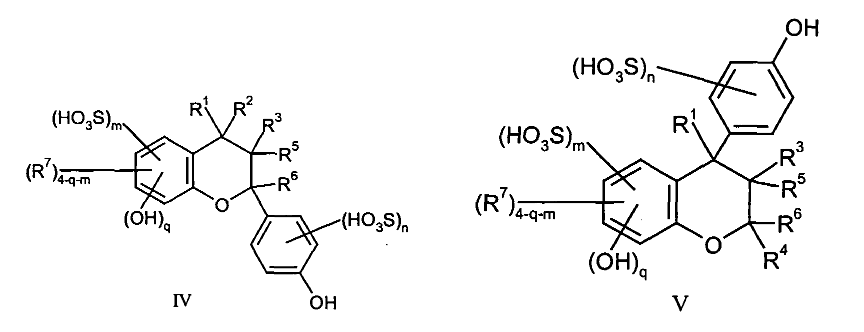 wobei
R¹ bis R⁶ soweit vorhanden, jeweils unabhängig voneinander für Wasserstoff, Alkyl, vorzugsweise C₁-C₄-Alkyl, insbesondere Methyl oder Aryl, vorzugsweise gegebenenfalls mit n Sulfogruppen sulfoniertes Hydroxyphenol stehen, bevorzugt für Wasserstoff, Methyl oder Hydroxyphenyl stehen, und
R⁷ für Wasserstoff oder den Rest 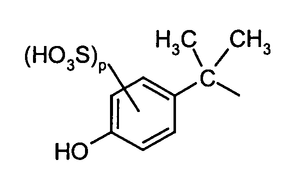
steht, wobei im Falle von mehreren Resten R⁷ diese Bedeutungen unabhängig von einander gelten,
m eine Zahl von 0 bis 3 bedeutet,
n eine Zahl von 0 bis 4 bedeutet,
p für eine Zahl von 0 bis 4 steht,
q für 0 oder 1 steht,
wobei die Summe aus m, n und p wenigstens für 1, bevorzugt 1,3 bis 4, besonders bevorzugt 1,8 bis 3,2 steht.

4. Verbindungen gemäß wenigstens einem der Ansprüche 1 bis 3, **dadurch gekennzeichnet, dass** sie in Form ihrer Säure der Formel IIa, IIIa, IVa oder Va entsprechen, 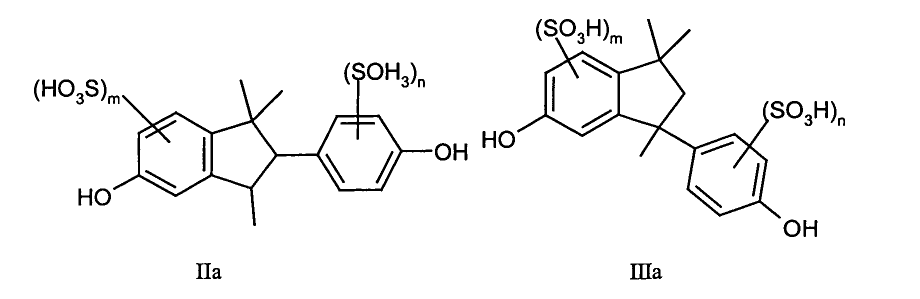 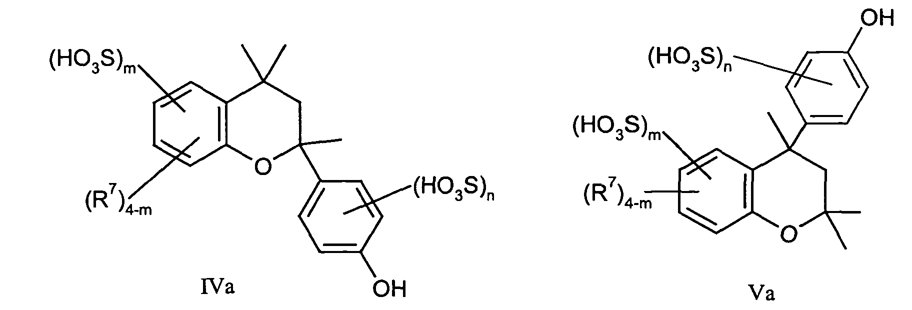 worin
R⁷ für Wasserstoff oder den Rest 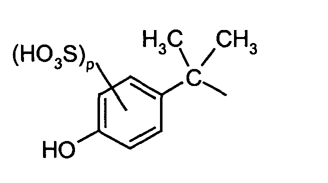
steht, wobei im Falle von mehreren Resten R⁷ diese Bedeutungen unabhängig voneinander gelten, und
m, n und p die oben angegebene Bedeutung besitzen.

5. Verbindungen gemäß wenigstens einem der Ansprüche 1 bis 4, wobei die Summe von n, m und p 1 bis 6, vorzugsweise 1,3 bis 4, besonders bevorzugt 1,8 bis 3,2 beträgt.

6. Verfahren zur Herstellung von Hydroxysulfonsäuren und/oder Hydroxysulfonaten nach wenigstens einem der Ansprüche 1 bis 5 **dadurch gekennzeichnet, dass** man Verbindungen der Formel VI 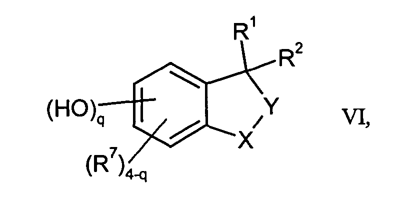 worin
X, Y, R¹, R², R⁷ und q die in einem der Ansprüche 1 bis 5 angegebenen Bedeutungen besitzen, und wenigstens ein R⁷ für Wasserstoff steht,
mit einem Sulfonierungsmittel sulfoniert.

7. Mischungen, enthaltend
| | |
|---|---|
| 0,5 bis 20 Gew.-% | der Verbindung der Formel II gemäß Anspruch 3 und |
| 0,5 bis 20 Gew.-% | der Verbindungen der Formel III gemäß Anspruch 3 und |
| 2 bis 25 Gew.-% | der Verbindungen der Formel IV gemäß Anspruch 3 und |
| 2 bis 25 Gew.-% | der Verbindungen der Formel V gemäß Anspruch 3 und |
| 0,5 bis 20 Gew.-% | sulfoniertes Phenol und |
| 10 bis 50 Gew.-% | sulfoniertes 4,4'-Isopropylidendiphenol und |
| 5 bis 40 Gew.-% | sulfoniertes o-[1-(4-Hydroxyphenyl)-1-methylethyl]phenol. |

8. Mischung nach Anspruch 7, wobei sie aus mehr als 80 Gew.-% aus den mit Mengenbereichen versehenen Komponenten besteht.

9. Aliphatische Aldehyd-Kondensate, insbesondere Formaldehyd-Kondensate auf Basis der Verbindungen nach wenigstens einem der Ansprüche 1 bis 5 oder der Mischung nach wenigstens einem der Ansprüche 7 bis 8.

10. Verwendung der Verbindungen gemäß wenigstens einem der Ansprüche 1 bis 5 oder Mischung nach 7 bis 8 oder der Kondensate nach Anspruch 9 zum Gerben von Häuten oder Fellen oder zum Nachgerben von mineralisch gegerbtem Leder, insbesondere chromgegerbtem Leder **dadurch gekennzeichnet, dass** die Häute oder Felle bzw. das gegerbte Leder mit den erfindungsgemäßen Stoffen in wässriger Flotte behandelt werden.

11. Leder oder Pelze, welche gegerbt oder nachgegerbt werden mit wenigstens einer Verbindung der Ansprüche 1 bis 5 oder 7 bis 8 oder 9.

12. Verwendung der Verbindungen gemäß wenigstens einem der Ansprüche 1 bis 5 oder 7 bis 8 oder der Kondensate nach Anspruch 9 als Hilfsmittel für die Papier- oder Textilanwendung.
